# EUROPEAN PATENT SPECIFICATION

(11) **EP 1 016 408 B1**
(45) Date of publication and mention of the grant of the patent: **19.04.2006**
(21) Application number: 98943009.5
(22) Date of filing: 16.09.1998
(51) Int. Cl.: A61K 31/557, C07D 307/93

(54) **NOVEL PAHARMACEUTICAL USE OF C-C CHEMOKINE PRODUCTION INHIBITORS**
NEUE PHARNAZEUTISCHE VERWENDUNG VON C-C CHEMOKIN PRODUKTIONSINHIBITOREN
NOUVELLE UTILISATION PHARMACEUTIQUE DES INHIBITEURS DE LA PRODUCTION DE CHEMOKINE C-C

(30) Priority: 16.09.1997 JP 25096697
(43) Date of publication of application: 05.07.2000
(73) Proprietor: TORAY INDUSTRIES, INC., Tokyo 103-8666 (JP)
(72) Inventor: KURUMATANI, Hajimu, Kamakura-shi, Kanagawa 248-0034 (JP); SASAKI, Rie, Fujisawa-shi, Kanagawa 251-0021 (JP); KUMAGAI, Hiroki, Kamakura-shi, Kanagawa 248-0034 (JP)
(74) Representative: Kador & Partner
(86) International application number: PCT/JP1998/004164
(87) International publication number: WO 1999/013880

(56) References cited:
- EP-A- 0 024 943
- EP-A- 0 060 640
- EP-A- 0 365 678
- JP-A- 7 188 205
- TANAKA H. et al., "The Effect of a synthetic 7-Thiaprostaglandin E1 Derivative TEI6122, on Monocyte Chemoattractant Protein-1 Induced Chemotaxis in THP-1 Cells", BRITISH JOURNAL OF PHARMACOLOGY, 1995, Vol. 116, No. 14, pages 2298-2302, XP002915324

## Description

### Technical Field

The present invention relates to a C-C chemokine production inhibitor comprising a prostanoic acid derivative as an active component.

### Background Art

In 1987, Matsushima et al. isolated IL-8 as a neutrophilic chemotactic factor from a culture supernant of human peripheral blood monocytes stimulated by a lipopolysaccharide, and then purified and cloned molecules having a migrating activity for many leukocytes. These molecules have a common structure, and are thus generically named "chemokine". Chemokine mainly has high affinity for heparin, and the common property that it is synthesized as a precursor composed of about 100 amino acids, and then secreted in a mature type comprising about 70 amino acids.

Chemokine generally has four cysteine residues, and is roughly classified into C-X-C chemokine comprising an amino acid held between the first two cysteine residues, and C-C chemokine having no amino acid between the cysteine residues. C-X-C chemokine is also called α chemokine, and C-C chemokine is called β chemokine. The C-C chemokine family is a generic name of a group of low-molecular-weight proteins having about 30% of homology in the amino acid level, and cysteine at the same four positions.

MCP-1 (Monocyte chemoatractant protein-1) is also named a monocyte chemotactive activating factor (MCCAF) or GDCF (glioma-derived monocyte chemotactic factor), and is a protein comprising 76 amino acids and four cysteine residues. The identification and gene cloning of MCAF, MCP-1 or GDCF have been reported (K. Matsushima et al., J. Exp. Med., 169, 1485-1490, 1989, Y. Furutani et al., Biochem. Biophys. Res. Commun., 159, 249-255, 1989, E. R. Robinson et al., Proc. Natl. Acad. Sci. USA, 86, 1850-1854, 1989, T. Yoshimura et al., FEBS Letters, 244, 487-493, 1989). These documents also disclose methods of producing MCP-1. In the present invention, MCP-1 is an abbreviated name and includes GDCF and MCAF hereinafter.

MCP-1 is produced from hemocytic cells such as monocytes, macrophages, and lymphocytes, as well as various cells such as fibroblasts, endothelial cells, smooth muscle cells, various tumor cells, and the like by stimulation with IL-1, TNF, IFN-, LPS, phorbol ester (TPA), or the like, and MCP-1 is known to cause accumulation of very strong monocytes and/or macrophages in a pathogenic region. MCP-1 also has a chemotactic action and activating action on basophils and T cells.

Known other proteins belonging to the C-C chemokine include RANTES, LD78, ACT2, I-309, MCP-2, MCP-3, JE, MIP-1α, MIP-1β, TCA-3, eotaxin, and the like.

Of these proteins, MCP-2, MCP-3 (K. B. M. Reid, Immunol. Today 10, 177-180, 1989), RANTES (P. N. Barlow et al., J. Mol. Biol. 232, 268-284, 1993), and JE (B. J. Rollins et al., Proc. Natl. Acad. Sci. USA, 85, 3738-3742, 1988) are known to have the inductive action to cause chemotaxis of monocytes and/or macrophages to a pathogenic region. RANTES also exhibits the strong chemotactic ability for basophils, eosinophils, and T-cells, and is related to chronic rheumatoid arthritis, endarterial hyperplasia after organ transplantation, rejection after organ transplantation, and allergic diseases.

MIP-1α is known to exhibit the chemotactic action on basophils, eosinophils, T-cells, B-cells, and NK-cells, and eotaxin has the strong chemotactic action on eosinophils.

Pathological progress of migration of eosinophils and basophils is frequently observed in acute serious inflammation, chronic intractable inflammation, bronchial asthma, allergic diseases, parasitic diseases, tumors, eosinophilic gastroantritis, peptic ulcer, valvular diseases, multiple sclerosis, osteoproresis, and organ re-perfusion disorder. Although migration of monocytes and macrophages to a pathogenic region is also observed in general inflammation, it is observed particularly in acute serious inflammation, chronic intractable inflammation, and allergic diseases, and also observed in nephritis, pneumonocirrhosis, arteriosclerosis, and malignant tumors.

It is known that diabetes highly frequently causes great vessel diseases such as arteriosclerosis, and microangiopathy causing complications such as diabetic neuropathy, diabetic nephropathy, diabetic retinopathy, and the like. However, it is thought to be important for the angiopathy that microphages are bonded to the endothelial cells and infiltrated into the vessel walls.

It is also known that in lung diseases, microphages are increased in the lung, and macrophages play an important role for fibrogenesis in the lung. Accumulation of macrophages is also observed in an affected part of chronic rheumatoid arthritis (RA).

Conventionally, a steroidal agent or non-steroidal anti-inflammatory agent is used for the above-described diseases. However, such medicines are known to suppress leukocyte migration, and at the same time, suppress the functions of many types of cells, thereby causing the problem of causing various serious side effects.

Prostaglandin (PG) includes a group of compounds naturally existing, exhibiting a variety of physiological activities, and having a common prostanoic acid skeleton. The natural PG compounds are classified into PGA, PGB, PGC, PGD, PGE, PGF, PGG, PGH, PGI, and PGJ by the structural characteristics of five-member rings, and further classified into sub-classes 1, 2, 3, etc. by the presence of unsaturation and oxidation. Also, many synthetic compounds analogous to these PG compounds are known. Of these PG compounds, a typical PGI derivative PGI₂ is referred to as "prostacyclin" (refer to Nature, Vol. 268, p688, 1976), and is known as a substance having strong platelet aggregation inhibiting action and peripheral vasodilating action. As compounds in which instability of the PGI₂ is significantly improved, Japanese Examined Patent publication Nos. 2-12226, 2-57548 and 1-53672 disclose PGI₂ derivatives having a skeleton in which the structure of an exoenol ether portion, which is a characteristic structure of PGI₂, is converted into an inter-m-phenylene type. Other known compounds in which stability of prostaglandin is improved include ataprost, iloprost, clinprost, ciprosteni, naxaprost, taprostene, cicaprost, pimilprost, CH-169, and CS570 (refer to Gendai-Iryosha, "Generals of Prostaglandin" No. 1, p. 123, 1994; New Drugs of Tomorrow, p. 15-IV-185, 1996; New Drugs of Tomorrow, p. 15-III-551, 1996). However, it is unknown that these prostanoic acid derivatives have the action to inhibit directly C-C chemokine production;

It is an object of the present invention to provide a preventive and curative medicine for diseases for which conventional medicines are ineffective, and which causes serious side effects and are characterized by abnormal accumulation or activation of leukocytes such as monocytes and/or macrophages, eosinophils, basophils, and the like.

### Disclosure of Invention

The present invention provides the use of a C-C chemokine production inhibitor as in claim 1.

### Brief Description of the Drawings

Fig. 1 shows the BPS action on MCP-1 production of THP-1 cells stimulated with LPS.
Fig. 2 shows the BPS action on the amount of MCP-1mRNA expression of THP-1 cells stimulated with LPS.
Fig. 3 shows the BPS action on MCP-1 production of human peripheral blood monocytes stimulated with LPS.
Fig. 4 shows the BPS action on MCP-3 production of THP-1 cells stimulated with LPS.
Fig. 5 shows the effect of BPS administration on the neurotransmission rate of streptozotocin-induced diabetic rats.
Fig. 6 shows the actions of various PG compounds on MCP-1 production of THP-1 cells stimulated with LPS.
Fig. 7 shows the effect of BPS administration on macrophage infiltration into glomeruli in a glomerulonephritis model (Fig. 7a), and on changes in renal gene expression (Fig. 7b).
Fig. 8 shows the effect of BPS administration on the amount of urine proteins in a glomerulonephritis model

As prostanoic acid derivates of the present inventions derivates of PGI2 or salts thereof are used. Specifically, 4,8-inter-m-phenylene prostaglandin I₂ derivatives represented by the following formula (I) or pharmacologically acceptable salts thereof are used. 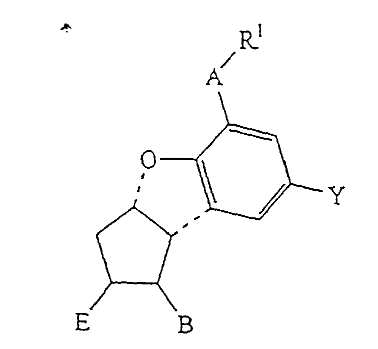 [wherein R¹ represents the following:
(A) COOR² wherein R² is:
   1) hydrogen or a pharmacologically acceptable cation;
   2) straight chain alkyl having 1 to 12 carbon atoms, or branched alkyl having 3 to 14 carbon atoms;
   3) -Z-R³
      wherein Z is a valence bond or straight chain or branched alkylene represented by CₜH₂ₜ wherein t represents an integer of 1 to 6, and R³ represents cycloalkyl having 3 to 12 carbon atoms or substituted cycloalkyl having 3 to 12, carbon atoms and 1 to 3 substituents R⁴ each of which is hydrogen or alkyl having 1 to 5 carbon atoms;
   4) -(CH₂CH₂O)ₙCH₃
      wherein n is an integer of 1 to 5;
   5) -Z-Ar¹
      wherein Z is defined as the same as the above, and Ar¹ is phenyl, α-naphthyl, β-naphthyl, 2-pyridyl, 3-pyridyl, 4-pyridyl, α-furyl, β-furyl, α-thienyl, β-thienyl or substituted phenyl (wherein a substituent is at least one of chlorine, fluorine, iodine, trifluoromethyl, alkyl having 1 to 4 carbon atoms, nitro, cyano, methoxy, phenyl, phenoxy, p-acetoamidobenzamide, -CH=N-NH-C(=O)-NH₂, -NH-C(=O)-Ph, -NH-C(=O)-CH₃ and -NH-C(=O)-NH₂);
   6) -CₜH₂ₜCOOR⁴
      wherein CₜH₂ₜ and R⁴ are defined as the same as the above;
   7) -CₜH₂ₜN(R⁴)₂
      wherein CₜH₂ₜ and R⁴ are defined as the same as the above;
   8) -CH(R⁵)-C(=O)-R⁶
      wherein R⁵ is hydrogen or benzoyl, and R⁶ is phenyl, p-bromophenyl, p-chlorophenyl, p-biphenyl, p-nitrophenyl, p-benzamidophenyl, or 2-naphthyl;
   9) -CₚH₂ₚ-W-R⁷
      wherein W is -CH=CH-, -CH=CR⁷ or -C C-, and R⁷ is hydrogen or straight chain or branched alkyl or aralkyl having 1 to 30 carbon atoms, and p is an integer of 1 to 5; or
   10) -CH(CH₂OR⁸)₂
      wherein R⁸ is alkyl or acyl having 1 to 30 carbon atoms;
(B) -CH₂OH;
(C) -C(=O)N(R⁹)₂
   wherein R⁹ is hydrogen, straight chain alkyl having 1 to 12 carbon atoms, branched alkyl having 3 to 12 carbon atoms, cycloalkyl having 3 to 12 carbon atoms, cycloalkylalkylene having 4 to 13 carbon atoms, phenyl, substituted phenyl (wherein the substituent is defined as the same as in (A) 5)), aralkyl having 7 to 12 carbon atoms, or -SO₂R¹⁰ wherein R¹⁰ is alkyl having 1 to 10 carbon atoms, cycloalkyl having 3 to 12 carbon atoms, phenyl, substituted phenyl (the substituent is defined as the same as in (A) 5)), or aralkyl having 7 to 12 carbon atoms, two R⁹ groups may be the same or different, and when one of the R⁹ groups is -SO₂R¹⁰, the other R⁹ is not SO₂R¹⁰; or
(D) -CH²OTHP (THP is a tetrahydropyranyl group);
   A is the following:
   1) -(CH₂)ₘ- ;
   2) -CH=CH-CH₂-;
   3) -CH₂-CH=CH-;
   4) -CH₂-O-CH₂-;
   5) -CH=CH-;
   6) -O-CH₂-; or
   7) -C C-;
   wherein m represents an integer of 1 to 3;
   Y is hydrogen, alkyl having 1 to 4 carbon atoms, chlorine, bromine, fluorine, formyl, methoxy or nitro;
   B is -X-C(R¹¹) (R¹²)OR¹³
   wherein R¹¹ is hydrogen, alkyl having 1 to 4 carbon atoms; R¹³ is hydrogen, acyl having 1 to 14 carbon atoms, aroyl having 6 to 15 carbon atoms, tetrahydropyranyl, tetrahydrofuranyl, 1-ethoxythienyl, or t-butyl; X is the following:
   1) -CH₂-CH₂-;
   2) -CH=CH-; or
   3) -C C-; and
   R¹² is the following:
   1) straight chain alkyl having 1 to 12 carbon atoms, or branched alkyl having 3 to 14 carbon atoms;
   2) -Z-Ar²
      wherein Z is defined as the same as the above, and Ar² represents phenyl, α-naphthyl, β-naphthyl, or phenyl substituted by at least one chlorine, bromine, fluorine, iodine, trifluoromethyl, alkyl having 1 to 4 carbon atoms, nitro, cyano, methoxy, phenyl or phenoxy;
   3) -CₜH₂ₜOR¹⁴
      wherein CₜH₂ₜ is defined as the same as the above, and R¹⁴ represents straight chain alkyl having 1 to 6 carbon atoms, branched alkyl having 3 to 6 carbon atoms, phenyl, phenyl substituted by at last one chlorine, bromine, fluorine, iodine, trifluoromethyl, alkyl having 1 to 4 carbon atoms, nitro, cyano, phenyl or phenoxy, cyclopentyl, cyclohexyl, or cyclopentyl or cyclohexyl substituted by 1 to 4 straight chain alkyl groups having 1 to 4 carbon atoms;
   4) -Z-R³
      wherein Z and R³ are defined as the same as the above;
   5) -CₜH₂ₜ-CH=C(R¹⁵)R¹⁶
      wherein CₜH₂ₜ is defined as the same as the above, and R¹⁵ and R¹⁶ each represent hydrogen, methyl, ethyl, propyl, or butyl; or
   6) -CᵤH₂ᵤ-C C-R¹⁷
      wherein u is an integer of 1 to 7, CᵤH₂ᵤ represents straight chain or branched alkyl, and R¹⁷ represents straight chain alkyl having 1 to 6 carbon atoms;
      E is hydrogen or -OR¹⁸
      wherein R¹⁸ represents acyl having 1 to 12 carbon atoms, aroyl having 7 to 15 carbon atoms, or R² (wherein R² is defined as the same as the above); and
      the formula represents a d, l or dl form].

Although preferred examples of prostaglandin I derivatives of the present invention include beraprost or salts thereof represented by the following formula (II), ataprost, iroprost, clinprost, ciprostene, naxaprostene, taprostene, cicaprost, pimilprost, CH-169, CS570, and the like, the prostaglandin I derivatives are not limited to these derivatives.

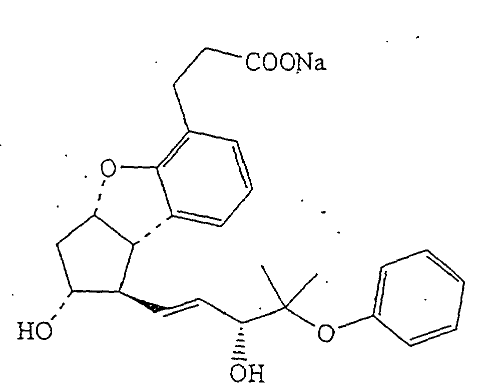

The prostanoic acid derivatives of the present invention can be produced by known methods. For example, compounds represented by formula (I) or salts thereof can be produced by the method disclosed in Japanese Examined Patent Publication No. 1-53672.

As described above, the prostanoic acid derivatives of the present invention inhibit the production of C-C chemokine which promotes migration of leukocytes to inhibit chemotaxis to a pathological region.

A typical example of C-C chemokine of the present invention is MCP-1. It has been reported that MCP-1 is produced from hemocytic cells such as monocytes, macrophages, and lymphocytes, as well as various cells such as fibroblasts, endothelial cells, smooth muscle cells, various tumor cells, and the like by stimulation with IL-1, TNF, IFN-γ, LPS, phorbol ester (TPA), or the like. Although other examples of C-C chemokine include RANTES, LD78, ACT2, I-309, MCP-2, MCP-3, JE, MIP-1α, MIP-1β, TCA-3, eotaxin, and the like, C-C chemokine compounds are not limited to these compounds.

As a result of detailed study of the actions of prostaonic acid derivatives, the inventors found that the compounds have the action to inhibit C-C chemoline production, leading to the achievement of the present invention. In the present invention, curable diseases are related to abnormal accumulation of leucocytes, particularly monocytes and/or magcrophages, eosinophils, basophils, and lymphocytes, accompanied with abnormal production of C-C chemokine. Such diseases include, chronic intractable inflammation, pneumonocirrhosis, pneumonia, ARDS, ulcerative colitis, chronic rheumatoid arthritis, atopic dermatitis, Crohn's disease, parasitic disease, and andometriosis.

Any one of the prostanoic acid derivatives of the present invention is administered 1 to 3 times a day in a dose of 0.001 to 1000 mg/adult. Although the C-C chemokine production inhibitor of the present invention may contain at least one prostanoic acid derivative, the inhibitor can also be orally administered in the form of a solid containing the additives below.

Examples of such additives include an excipient such as starch, lactose, sucrose, glucose, mannitol, potassium carbonate, calcium sulfate, or the like; a binder such as starch, dextrin, gum arabic, tragacanth, methyl cellulose, gelatin, polyvinyl pyrrolidone, polyvinyl alcohol, or the like; a disintegrator such as starch, polyvinyl pyrrolidone, crystalline cellulose, or the like; a lubricant such as magnesium stearate, talc, or the like; a colorant; a flavor; and the like.

The prostanoic acid derivatives of the present invention can be used in various forms. Examples of the forms include conventional forms such as a tablet, a sugar-coated tablet, a powder, granules, a troche, a capsule, a pill, a syrup, a spray, and the like. The derivatives may also be parenterally administered in the form of a sterilized solution, and another solute such as sodium chloride, glucose, or the like can also be used in an amount sufficient for making the solution isotonic. The C-C chemokine production inhibitor of the present invention can be applied to the above-described oral administration as well as parenteral administration of injections, suppositories, etc.

### [Examples]

The present invention will be described in detail below with reference to examples.

### Example 1

### Action on amount of MCP-1 production of human monocyte/macrophage system cells THP-1:

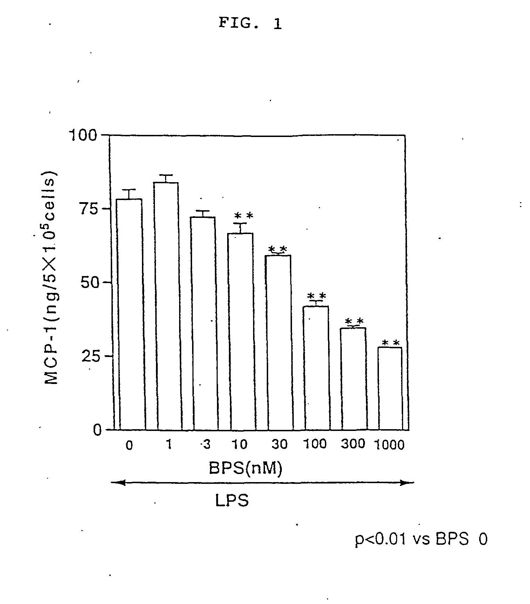

The action of beraprost (BPS) on MCP-1 production was examined by using human monocyte/macrophage system leukemic cells THP-1. Lipopolysaccharide (LPS: Difco Corp.) reactive substrains were isolated from THP-1 cells (obtained from ATCC Corp.), and cultured in a RPMI1640 medium (Gibco Corp.) containing 10% FCS in a flask. The THP-1 cells (1 x 10⁵ cells) were dispensed to a 12-well plate, and activated with 10 µg/ml of LPS. BPS was added 5 minutes before LPS stimulation. A cell supernatant was obtained 24 hours after stimulation, and the amount of MCP-1 production was measured by using a human MCP-1 eraser kit (R&D Corp.). The amount of production was calculated based on a calibration curve formed in the range of 31.2 to 2000 pg/ml by using MCP-1 standards contained in the kit. The results indicate that BPS inhibits dose-dependently the production of monocyte chemotactic factor of the THP-1 cells induced by 10 µg/ml of LPS (Fig. 1).

### Example 2

### Action on expression of MCP-1m-RNA of human monocyte/macrophage system cells THP-1:

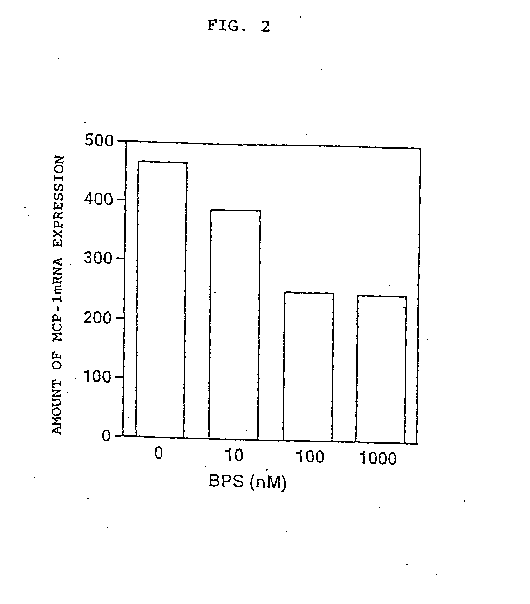

The action of BPS on MCP-1 production was examined by using human monocyte/macrophage system leukemic cells THP-1 and the amount of mRNA expression as an index. LPS reactive substrains were isolated from THP-1 cells, and cultured in a RPMI1640 medium containing 10% FCS in a flask. THP-1 cells (1 x 10⁶ cells) were dispensed to a petri dish having a diameter of 10 cm, and activated with 10 µg/ml of LPS. BPS was added 5 minutes before LPS stimulation. The total of RNA was extracted with a LiCl-urea solution (6M urea/3M LiCl/5mM EDTA) 24 hours after stimulation, and dissolved in a TE solution (10 mM Tris-HCl/1mM EDTA, pH 8.0), and then proteins were removed with phenol and chloroform, followed by RNA recovery by ethanol precipitation. RNA was developed by formaldehyde-modified 1% agarose gel, and transferred to a Hybond-N filter (Amersham Corp.), and then MCP-1mRNA was detected by using a ³²P-labeled human MCP-1 probe. The detection was carried out by using a X-ray film or imaging film, and the amount of expression was digitized by BAS2000. The results indicate that BPS inhibits dose-dependently expression of MCP-1mRNA of THP-1 cells stimulated by LPS (Fig. 2).

### Example 3

### Action on MCP-1 production of human peripheral blood-derived monocytes

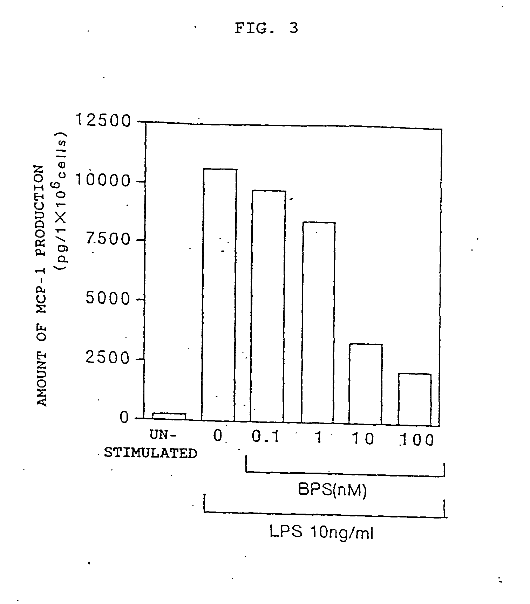

The action of BPS on MCP-1 production was studied by using human peripheral blood-derived monocytes. The heparinized peripheral blood of a health person was superposed on a histo-pack (Sigma Corp.), and subjected to a centrifugal operation to obtain a monocyte layer. The thus-obtained monocyte layer was reacted with magnetic beads (Miltenyi Biotec Corp.) of anti-CD3 and anti-CD19, and monocytes were purified by a negative selection method using a MACS column (Miltenyi Biotec Corp.). The thus-obtained monocytes were re-suspended in a RPMI 1640 medium so that 1 x 10⁶ cells/ml of cells were obtained. The cells were dispensed to a 48-well plate, and activated with 10 ng/ml of LPS. BPS was added 5 minutes before LPS stimulation. A cell supernatant was obtained 24 hours after stimulation, and the amount of the monocyte chemotactic factor produced was measured by using a human MCP-1 eraser kit. The amount of production was calculated based on a calibration curve formed in the range of 31.2 to 2000 pg/ml by using MCP-1 standards contained in the kit. The results indicate that BPS inhibits dose-dependently the production of MCP-1 cells of human peripheral blood monocytes induced by 10 ng/ml of LPS (Fig. 3).

### Example 4

### Action on production of MCP-3 of human monocyte/macrophage system cells THP-1

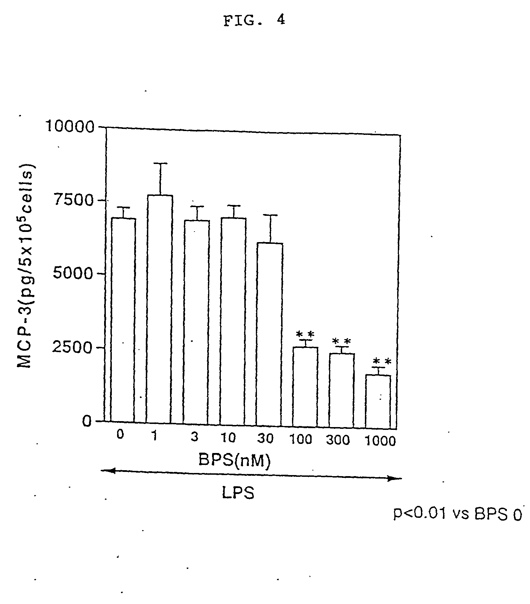

The action of BPS on MCP-3 production was examined by using human monocyte/macrophage system leukemic cells THP-1. LPS reactive substrains were isolated from THP-1 cells, and cultured in a RPMI1640 medium containing 10% FCS in a flask. THP-1 cells (1 x 10⁶ cells) were dispensed to a 12-well plate, and activated with 10 µg/ml of LPS. BPS was added 5 minutes before LPS stimulation. A cell supernatant was obtained 24 hours after stimulation, and the amount of the MCP-3 cells produced was measured by using a MCP-3 detection method developed by the inventors. The amount of production was calculated based on a calibration curve formed in the range of 0.195 to 12.5 ng/ml by using MCP-3 standards. The results indicate that BPS inhibits dose-dependently not only the production of THP-1 cells but also the production of MCP-3 cells induced by 10 µg/ml of LPS (Fig. 4).

### Example 5

### Effect of BPS administration on MCP-1 amount in LPS-induced blood of diabetic rats .

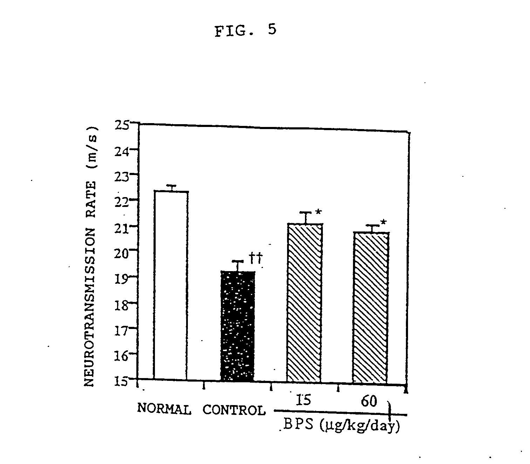

SD male rats were intravenously administered with 45 mg/kg streptozotocin to induce diabetes. At the eighth week after induction, 2 mg/kg of LPS was administered to the rats to measure the MCP-1 amount in the blood before administration and 3 and 6 hours after administration. BPS was orally administered to the rats 30 minutes before LPS administration. Rats of the same week old as the diabetic rat group were used as a normal group. The results are shown in Table 1. In the diabetic rats, MCP-1 production was significantly progressed by administering LPS, as compared with the normal rats. In the rat group administered with BPS, MCP-1 production was significantly inhibited, thereby indicating in in-vivo experiment that abnormal production of MCP-1 due to diabetes is improved by BPS. As a result of measurement of the neurotransmission rate of the ischiatic nerve by using the same diabetic rats, a decrease in the neurotransmission rate due to diabetes was significantly improved by BPS (Fig. 5).

**Table 1 Effect of Oral Administration of BPS on MCP-1 amount in LPS-Induced Blood of Rats Administered with Streptozotocin**

| Treatment | n | MCP-1 amount in blood (ng/ml) Before LPS 3 hours after 6 hours after administration | | |
|---|---|---|---|---|
| Normal group | 4 | 0.30±0.11 | 7.39±1.22‡‡ | 3.86±1.44‡ |
| Diabetic group Untreated | 4 | 0.30±0.04 | 13.47±4.40# | 9.42±1.59 |
| BPS administered | 3 | 0.39±0.13 | 7.27±2.55‡‡ | 4.82±4.40‡ |

| | | | | |
|---|---|---|---|---|
| Numerals represent average ± standard deviation | | | | |
| ‡: p < 0.05, ‡‡: p < 0.01 in comparison with the untreated diabetic group (Student's test) | | | | |
| #:the value obtained from 3 samples because of defects in a sample. | | | | |

### Example 6

### Action on MCP-1 production of human monocyte/macrophage system cells THP-1

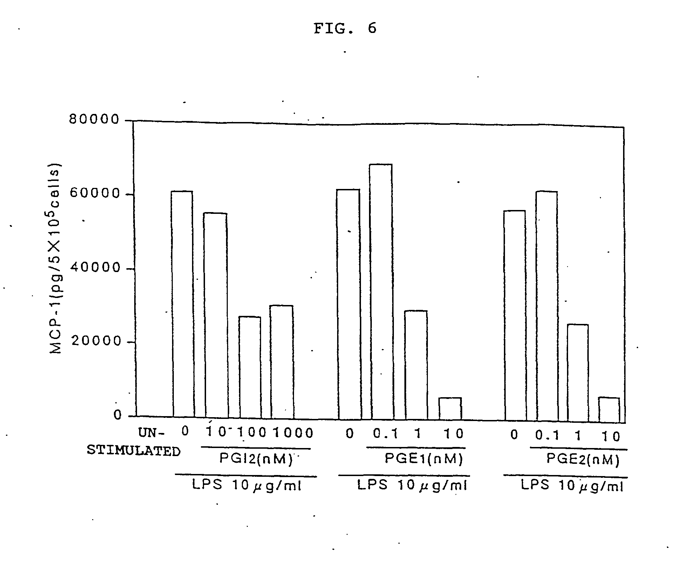

The actions of prostaglandin I₂ (PGI₂), prostaglandin E₁ (PGE₁), and prostaglandin E₂ (PGE₂) on MCP-1 production were examined by the same method as Example 1. As a result, MCP-1 production was inhibited by PGI₂, PGE₁, and PGE₂ (Fig. 6).

### Example 7

### Action on MCP-1 production of human monocyte/macrophage system cells THP-1

The actions of the compounds shown in the table below on MCP-1 production by the same method as Example 1. The action of each of the compounds was shown by an inhibition rate. As a result, MCP-1 production was inhibited by these compounds (Table 2).

### Example 8

### Action on MCP-1 production of monocytes derived from human peripheral blood

Monocytes derived from the human peripheral blood were isolated by the same method as Example 3, and stimulated with 25 nM 12-o-tetradecanoylphorbol 13-acetate (TPA) to induce MCP-1 production. The action of BPS on MCP-1 production was studied. As a result, 224.7 pg/1 x 10⁶ cells of MCP-1 produced by TPA stimulation was decreased to 184 pg/1 x 10⁶ cells by 100 nM BPS, and thus it was confirmed that MCP-1 production due to TPA stimulation is inhibited by BPS.

### Example 9

### Action on MCP-1 production of monocytes derived from human peripheral blood

The action of prostaglandin J₂ (PGJ₂) on MCP-1 production due to LPS or TPA simulation was studied by the same method as Examples 3 and 8 using monocytes derived from the human peripheral blood. PGJ₂ was added 1 minute before stimulation. As a result, MCP-1 production due to stimulation by either LPS or TPA was inhibited by PGJ₂ (Table 3).

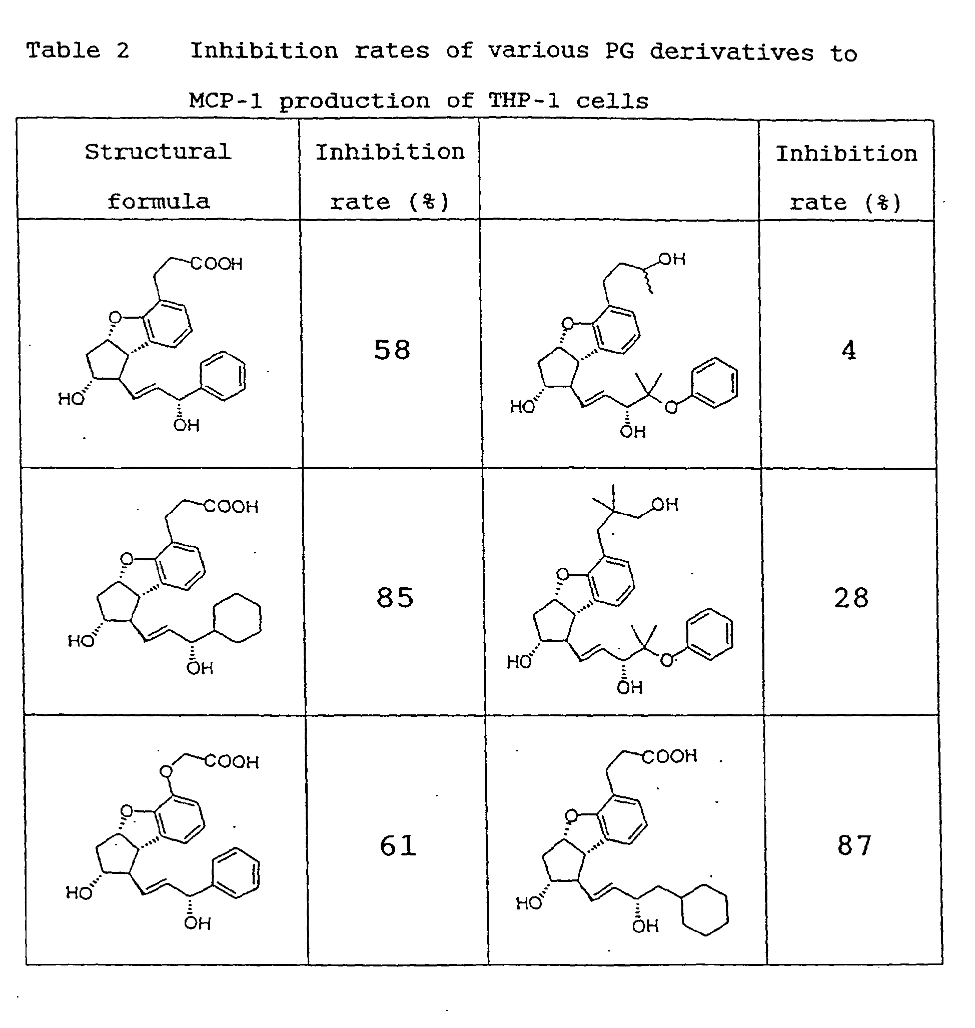

**Table 3 Action of PGJ2 on MCP-1 production of human peripheral blood monocytes**

| Treatment | MCP-1 (pg/10⁶ cells) |
|---|---|
| LPS 10 ng/ml | 21872 |
| LPS 10 ng/ml + PGJ2 10 µM | 6 |
| TPA 25 nM | 225 |
| TPA 25 nM + PGJ2 10 µM | 2 |

### Example 10

### Action on RANTES production of human monocyte/macrophage system cells THP-1

THP-1 cells were prepared by the same method as Example 1, and stimulated with LPS to induce RANTES production. The amount of RANTES production was measured by RANTES eraser kit (R&D Corp.). The action of PGJ₂ on RANTES production was studied. As a result, 5177 pg/5 x 10⁵ cells of RANTES produced by LPS stimulation was decreased to 2403 pg/5 x 10⁵ cells by 10 µM PGJ₂, and it was thus confirmed that RANTES production due to LPS stimulation is inhibited by PGJ₂.

### Example 11

### Action on MCP-1 production and macrophage infiltration in kidney in glomerulonephritis rat model:

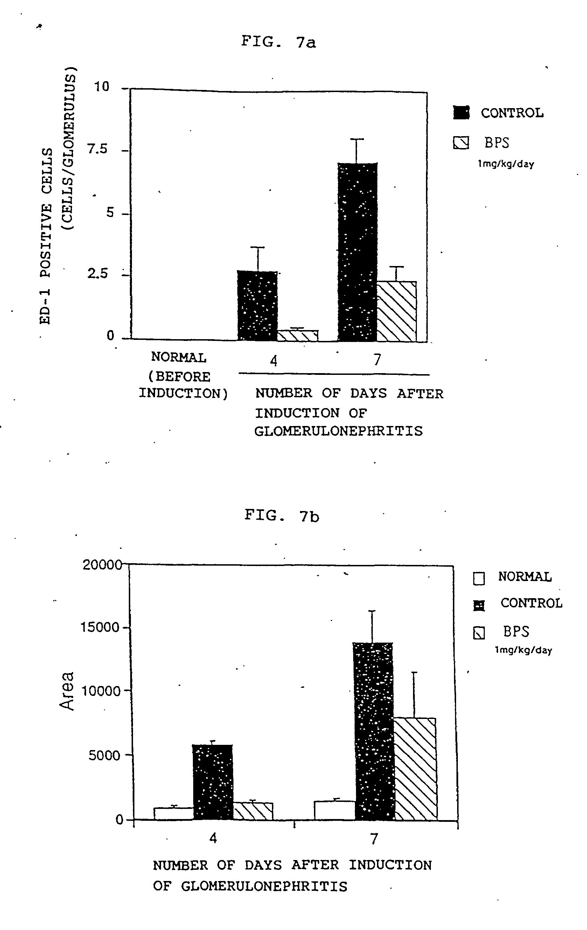

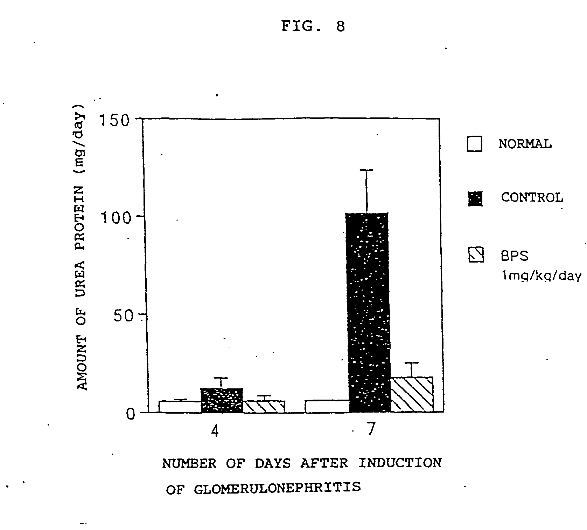

A glomerular basement membrane was administered with an antibody to form a glomerulonephritis rat model. The rats used were male WKY rats of 9 week old which were purchased from Japan Charles River. The antibody was obtained by immunizing the rat glomerular basement membrane against rabbits. The amount of proteins in urea was increased 4 days after administration of the antibody, reached to a plateau 11 days after administration, and then did not change up to the death of the rats. A glomerular lesion such as formation of the crescent was also confirmed by pathological findings, and an irreversible glomerulonephritis model could be formed by administering the antibody to the anti-glomerular basement membrane. MCP-1 production in the kidney was increased with the passage of days after administration of the antibody to produce infiltration of leukocytes such as macrophages and the like. 1 mg/kg of BPS was orally administered on consecutive days to study the action of BPS on MCP-1 production and infiltration of the macrophages. The MCP-1 production was studied by purifying messenger RNA (mRNA) from the rat kidneys, and determining the amount of MCP-1mRNA expression by quantitative PCR. The macrophage infiltration was studied by immunostaining with an anti-macrophage antibody (ED-1), and then counting the macrophages infiltrated into the glomeruli under a microscope. As a result of comparison between a group administered with distilled water and a group administered with BPS 4 days and 7 days after administration of the antibody, in the group administered with BPS, infiltration of macrophage per glomerulus was significantly inhibited in parallel with inhibition of MCP-1mRNA expression (Figs. 7a and b). Using the same glomerulonephritis model, the action of BPS on the amount of urea proteins was studied 4 days and 7 days after administration of the antibody. As a result, the amount of protein was significantly decreased by BPS both 4 days and 7 days after the administration (Fig. 8).

### Industrial Applicability

Prostaglandin derivatives have the action to inhibit C-C chemokine production, and are effective to cure circulatory diseases, inflammation, allergic diseases, renal diseases, etc.

## Claims

1. Use of a C-C chemokine production inhibitor for the manufacture of a medicament for chronic intractable inflammation, pneumonocirrhosis, pneumonia, ARDS, ulcerative colitis, chronic rheumatoid arthritis, atopic dermatititis, Crohn's disease, parasitic diseases and endometriosis, wherein the prostaglandin I₂ derivative is a 4,8-inter-m-phenylene prostaglandin I₂ derivative represented by the following formula, or pharmalogically acceptable salt thereof: 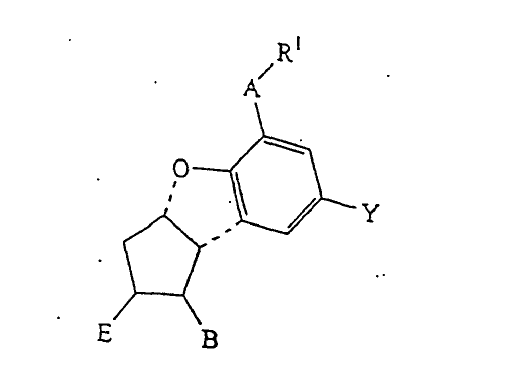 [wherein R¹ represents the following:
(A) COOR² wherein R² is:
1) hydrogen or a pharmacologically acceptable cation;
2) straight chain alkyl having 1 to 12 carbon atoms, or branched alkyl having 3 to 14 carbon atoms;
3) -Z-R³
wherein Z is a valence bond or straight chain or branched alkylene represented by CₜH₂ₜ wherein t represents an integer of 1 to 6, and R³ represents cycloalkyl having 3 to 12 carbon atoms or substituted cycloalkyl having 3 to 12 carbon atoms and 1 to 3 substituents R⁴ each of which is hydrogen or alkyl having 1 to 5 carbon atoms;
4) -(CH₂CH₂O)ₙCH₃
wherein n is an integer of 1 to 5;
5) -Z-Ar¹
wherein Z is defined as the same as the above, and Ar¹ is phenyl, α-naphthyl, β-naphthyl, 2-pyridyl, 3-pyridyl, 4-pyridyl, α-furyl, β-furyl, α-thienyl, β-thienyl or substituted phenyl (wherein a substituent is at least one of chlorine, fluorine, iodine, trifluoromethyl, alkyl having 1 to 4 carbon atoms, nitro, cyano, methoxy, phenyl, phenoxy, p-acetoamidobenzamide, -CH=N-NH-C(=O)-NH₂, -NH-C(=O)-Ph, -NH-C(=O)-CH₃ and -NH-C(=O)-NH₂);
6) -CₜH₂ₜCOOR⁴
wherein CₜH₂ₜ and R⁴ are defined as the same as the above;
7) -CₜH₂ₜN(R⁴)₂
wherein CₜH₂ₜ and R⁴ are defined as the same as the above;
8) -CH(R⁵)-C(=C))-R⁶
wherein R⁵ is hydrogen or benzoyl, and R⁶ is phenyl, p-bromophenyl, p-chlorophenyl, p-biphenyl, p-nitrophenyl, p-benzamidophenyl, or 2-naphthyl;
9) -CₚH₂ₚ-W-R⁷
wherein W is -CH=CH-, -CH=CR⁷ or -C C-, and R⁷ is hydrogen or straight chain or branched alkyl or aralkyl having 1 to 30 carbon atoms, and p is an integer of 1 to 5; or
10) -CH(CH₂OR⁸)₂
wherein R⁸ is alkyl or acyl having 1 to 30 carbon atoms;
(B) - CH₂OH ;
(C) -C(=O)N(R⁹)₂
wherein R⁹ is hydrogen, straight chain alkyl having 1 to 12 carbon atoms, branched alkyl having 3 to 12 carbon atoms, cycloalkyl having 3 to 12 carbon atoms, cycloalkylalkylene having 4 to 13 carbon atoms, phenyl, substituted phenyl (wherein the substituent is defined as the same as in (A) 5)), aralkyl having 7 to 12 carbon atoms, or -SO₂R¹⁰ wherein R¹⁰ is alkyl having 1 to 10 carbon atoms, cycloalkyl having 3 to 12 carbon atoms, phenyl, substituted phenyl (the substituent is defined as the same as in (A) 5)), or aralkyl having 7 to 12 carbon atoms, two R⁹ groups may be the same or different, and when one of the R⁹ groups is -SO₂R¹⁰, the other R⁹ is not -SO₂R¹⁰; or
(D) -CH²OTHP (THP is a tetrahydropyranyl group);
A is the following:
1) -(CH₂)ₘ-;
2) -CH=CH-CH₂-;
3) -CH₂-CH=CH-;
4) -CH₂-O-CH₂-;
5) -CH=CH-;
6) -O-CH₂-; or
7) -C C-;
wherein m represents an integer of 1 to 3;
Y is hydrogen, alkyl having 1 to 4 carbon atoms, chlorine, bromine, fluorine, formyl, methoxy or nitro;
B is -X-C(R¹¹)(R¹²)OR¹³
wherein R¹¹ is hydrogen, alkyl having 1 to 4 carbon atoms; R¹³ is hydrogen, acyl having 1 to 14 carbon atoms, aroyl having 6 to 15 carbon atoms, tetrahydropyranyl, tetrahydrofuranyl, 1-ethoxythienyl, or t-butyl; X is the following:
1) -CH₂-CH₂-;
2) -CH=CH-; or
3) -C C-; and
R¹² is the following:
1) straight chain alkyl having 1 to 12 carbon atoms, or branched alkyl having 3 to 14 carbon atoms;
2) -Z-Ar²
wherein Z is defined as the same as the above, and Ar² represents phenyl, α-naphthyl, β-naphthyl, or phenyl substituted by at least one chlorine, bromine, fluorine, iodine, trifluoromethyl, alkyl having 1 to 4 carbon atoms, nitro, cyano, methoxy, phenyl or phenoxy;
3) -CₜH₂ₜOR¹⁴
wherein CₜH₂ₜ is defined as the same as the above, and R¹⁴ represents straight chain alkyl having 1 to 6 carbon atoms, branched alkyl having 3 to 6 carbon atoms, phenyl, phenyl substituted by at last one chlorine, bromine; fluorine, iodine, trifluoromethyl, alkyl having 1 to 4 carbon atoms, nitro, cyano, phenyl or phenoxy, cyclopentyl, cyclohexyl, or cyclopentyl or cyclohexyl substituted by 1 to 4 straight chain alkyl groups having 1 to 4 carbon atoms;
4) -Z-R³
wherein Z and R³ are defined as the same as the above;
5) -CₜH₂ₜ-CH=C(R¹⁵)R¹⁶
wherein CₜH₂ₜ is defined as the same as the above, and R¹⁵ and R¹⁶ each represent hydrogen, methyl, ethyl, propyl, or butyl; or
6) -CᵤH₂ᵤ-C C-R¹⁷
wherein u is an integer of 1 to 7, CᵤH₂ᵤ represents straight chain or branched alkyl, and R¹⁷ represents straight chain alkyl having 1 to 6 carbon atoms;
E is hydrogen or -OR¹⁸
wherein R¹⁸ represents acyl having 1 to 12 carbon atoms, aroyl having 7 to 15 carbon atoms, or R² (wherein R² is defined as the same as the above); and
the formula represents a d, l or dl form].

2. Use according to Claim 1, wherein the 4,8-inter-m-phenylene prostaglandin I₂ derivative is beraprost or a salt thereof.

3. Use according to Claims 1 or 2, wherein C-C chemokine is MCP-1 or MCP-3.

4. Use according to Claims 1 to 3, wherein diseases which can be cured by a c-c chemokine production inhibitor are diseases **characterized by** abnormal accumulation or activation of leukocytes.

5. Use according to Claims 1 to 4, wherein leukocytes showing abnormal accumulation or activation are monocytes and/or macrophages.

6. Use according to Claims 1 to 5, wherein leukocytes showing abnormal accumulation or activation comprise at least one type of eorinophils, basophils, and lymphocytes.

## Patentansprüche

1. Verwendung eines Inhibitors der C-C-Chemokinproduktion zur Herstellung eines Medikaments gegen chronisch hartnäckige Entzündung, Pneumonozirrhose, Lungenentzündung, akute respiratorische Insuffizienz, eitrige Colitis, chronische rheumatische Arthritis, atopische Dermatitis, Crohn'sche Krankheit, parasitäre Krankheiten und Endometriose, wobei das Prostaglandin-I₂-Derivat ein 4,8-inter-m-Phenylenprostaglandin-I₂-Derivat ist, das durch die folgende Formel oder ein pharmakologisch akzeptables Salz davon wiedergegeben wird: 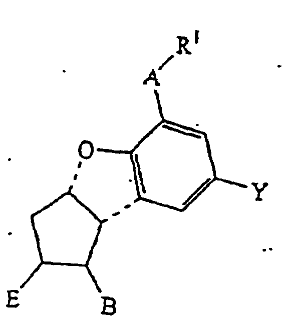 [wobei R¹ folgendes darstellt:
(A) COOR², wobei R²
1) Wasserstoff oder ein pharmakologisch akzeptables Kation;
2) ein geradkettiger Alkylrest mit 1 bis 12 Kohlenstoffatomen oder ein verzweigter Alkylrest mit 3 bis 14 Kohlenstoffatomen;
3) ―Z-R³
wobei Z eine Valenzbindung oder ein geradkettiger oder verzweigter Alkylenrest, der durch CₜH₂ₜ repräsentiert wird, wobei t eine ganze Zahl von 1 bis 6 und R³ einen Cycloalklrest mit 3 bis 12 Kohlenstoffatomen oder einen substituierten Cycloalkylrest mit 3 bis 12 Kohlenstoffatomen und 1 bis 3 Substituenten R⁴ darstellt, von denen jeder einzelne Wasserstoff der ein Alkylrest mit 1 bis 5 Kohlenstoffatomen ist;
4) -CH₂CH₂O)ₙCH₃
wobei n eine ganze Zahl von 1 bis 5 ist;
5) -Z-Ar¹
wobei Z wie oben definiert ist und Ar¹ ein Phenyl-, α-Naphthyl-, β-Naphthyl-, 2-Pyridyl-, 3-Pyridyl-, 4-Pyridyl-, α-Furyl-, β-Furyl-, α-Thienyl-, β-Thienyl- oder substituierter Phenylrest (wobei ein Substituent wenigstens einer der folgenden ist: Chlor, Fluor, Brom, Iod, Trifluormethyl, Alkyl mit 1 bis 4 Kohlenstoffatomen, Nitro, Cyano, Methoxy, Phenyl, Phenoxy, p-Acetoamidobenzamid, -CH=N-NH-C(=O)-NH₂, -NH-C(=O)-NH₂);
6) -CₜH₂ₜCOOR⁴
wobei CₜH₂ₜ und R⁴ definiert werden wie oben;
7) -CₜH₂ₜN(R⁴)₂
wobei CₜH₂ₜ und R⁴ definiert werden wie oben;
8) -CH(R⁵)-C(=O)-R⁶
wobei R⁵ Wasserstoff oder Benzoyl ist und R⁶ Phenyl, p-Bromphenyl, p-Chlorphenyl, p-Biphenyl, p-Nitrophenyl, p-Benzamidophenyl oder 2-Naphthyl ist;
9) -CₚH₂ₚ-W-R⁷
wobei W -CH=CH-, -CH=CR⁷ oder -C=C- ist und R⁷ Wasserstoff oder ein geradkettiger oder verzweigter Alkyl-oder Aralkylrest mit 1 bis 30 Kohlenstoffatomen ist und p eine ganze Zahl von 1 bis 5 ist;
oder
10) -CH(CH₂OR⁸)₂
wobei R⁸ ein Alkyl- oder Acylrest mit 1 bis 30 Kohlenstoffatomen ist;
(B) -CH₂OH;
(C) -C(=O)N(R⁹)₂
wobei R⁹ Wasserstoff, ein geradkettiger Alkylrest mit 1 bis 12 Kohlenstoffatomen, ein verzweigter Alkylrest mit 3 bis 12 Kohlenstoffatomen, ein Cycloalkylalkylen mit 4 bis 13 Kohlenstoffatomen, ein Phenyl, ein substituiertes Phenyl (wobei der Substituent definiert ist wie unter (A) 5)), ein Aralkyl mit 7 bis 12 Kohlenstoffatomen oder -SO₂R¹⁰ ist, wobei R¹⁰ ein Alkyl mit 1 bis 10 Kohlenstoffatomen, ein Cycloalkyl mit 3 bis 12 Kohlenstoffatomen, Phenyl, substituiertes Phenyl (der Substituent ist wie unter (A) 5) definiert) oder Aralkyl mit 7 bis 12 Kohlenstoffatomen ist, zwei R⁹-Gruppen gleich oder verschieden sein können und wenn eine der R⁹-Gruppen -SO₂R¹⁰, die andere R⁹-Gruppe nicht -SO₂R¹⁰ ist; oder
(D) -CH₂OTHP (THP ist Tetrahydropyranyl);
A ist folgendes:
1) -(CH₂)m-;
2) -CH=CH-CH₂-;
3) -CH₂-CH=CH-;
4) -CH₂-O-CH₂-;
5) -CH=H-;
6) ―O-CH₂-; oder
7) -C=C-;
wobei m eine ganze Zahl von 1 bis 3 darstellt;
Y Wasserstoff, ein Alkyl mit 1 bis 4 Kohlenstoffatomen, Chlor, Brom, Fluor, Formyl, Methoxy oder Nitro ist;
B -X-C(R¹¹)(R¹²)OR¹³ ist, wobei R¹¹ Wasserstoff, ein Alkyl mit 1 bis 4 Kohlenstoffatomen; R¹³ ein Wasserstoff, ein Acylrest mit 1 bis 14 Kohlenstoffatomen, ein Aroylrest mit 6 bis 15 Kohlenstoffatomen, Tetrahydropyranyl, Tetrahydrofuranyl, 1-Ethoxythienyl oder t-Butyl ist; X ist folgendes:
1) -CH₂-CH₂-;
2) -CH=CH-; oder
3) -C≡C-; und
R¹² folgendes ist:
1) ein geradkettiger Alkylrest mit 1 bis 12 Kohlenstoffatomen oder ein verzweigter Alkylrest mit 3 bis 14 Kohlenstoffatomen;
2) -Z-Ar²
wobei Z definiert ist wie oben und Ar² Phenyl, α-Naphthyl, β-Naphthyl oder ein mit wenigstens einem Chlor, Brom, Fluor, Iod, Trifluormethyl, einem Alkylrest mit 1 bis 4 Kohlenstoffatomen, Nitro, Cyano, Methoxy, Phenyl oder Phenoxy substituierten Phenylrest darstellt;
3) -CₜH₂ₜOR¹⁴
wobei CₜH₂ₜ definiert ist wie oben und R¹⁴ einen geradkettigen Alkylrest mit 1 bis 6 Kohlenstoffatomen, einen verzweigten Alkylrest mit 3 bis 6 Kohlenstoffatomen, Phenyl, einen mit wenigstens einem Chlor, Brom, Fluor, Iod, Trifluormethyl, einem Alkylrest mit 1 bis 4 Kohlenstoffatomen, Nitro, Cyano, Phenyl, Phenoxy, Cyclopentyl, Cyclohexyl oder mit 1 bis 4 geradkettigen Alkylresten mit 1 bis 4 Kohlenstoffen subtituiertes Cyclopentyl oder Cyclohexyl substierten Phenylrest darstellt;
4) -Z-R³
wobei Z und R³definiert werden wie oben;
5) ―CₜH₂ₜ-CH=C(R¹⁵)R¹⁶
wobei CₜH₂ₜ definiert ist wie oben und R¹⁵ und R¹⁶ jeweils Wasserstoff, Methyl, Ethyl, Propyl oder Butyl darstellen; oder
6) -CᵤH₂ᵤ-C≡CR¹⁷
wobei u eine ganze Zahl von 1 bis 7, CᵤH₂ᵤ einen geradkettigen oder verzweigten Alkylrest und R¹⁷ einen geradkettigen Alkylrest mit 1 bis 6 Kohlenstoffatomen darstellt;
E ist Wasserstoff oder -OR¹⁸
wobei R¹⁸ einen Acylrest mit 1 bis 12 Kohlenstoffatomen, einen Aroylrest mit 7 bis 15 Kohlenstoffatomen oder R² (wobei R² wie oben definiert ist); und
die Formel eine d-, 1- oder dl-Form darstellt].

2. Verwendung gemäß Anspruch 1, wobei das 4,8-inter-m-Phenylenprostaglandin-I₂-Derivat Beraprost oder eines dessen Salze ist.

3. Verwendung gemäß Anspruch 1 oder 2, wobei das C-C-Chemokin MCP-1 oder MCP-3 ist.

4. Verwendung gemäß Ansprüchen 1 bis 3, wobei die durch den Inhibitor der C-C-Chemokinproduktion behandelbaren Krankheiten **gekennzeichnet sind durch** abnormale Anhäufung oder Aktivierung von Leukozyten.

5. Verwendung gemäß Ansprüchen 1 bis 4, wobei die Leukozyten, die abnormale Anhäufung oder Aktivierung zeigen, Monozyten und/oder Makrophagen sind.

6. Verwendung gemäß Ansprüchen 1 bis 5, wobei die Leukozyten, die abnormale Anhäufung oder Aktivierung zeigen, mindestens einen Typ Eosinophile, Basophile und Lymphozyten umfassen.

## Revendications

1. Utilisation d'un inhibiteur de production de chimiokine C-C pour la fabrication d'un médicament pour l'inflammation réfractaire chronique, la fibrose pulmonaire, la pneumonie, le SDRA, la colite ulcéreuse, la polyarthrite rhumatoïde chronique, la dermatite atopique, la maladie de Crohn, les maladies parasitaires et l'endométriose, dans laquelle le dérivé de prostaglandine I₂ est un dérivé de 4,8-inter-m-phénylène prostaglandine I₂ représenté par la formule suivante, ou l'un de ses sels pharmacologiquement acceptables : 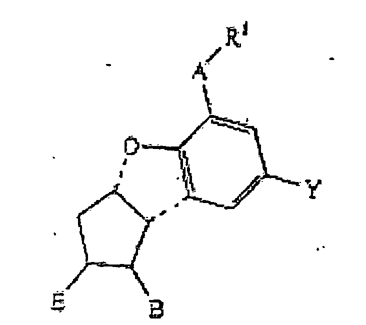 [dans laquelle R¹ représente les radicaux suivants :
(A) COOR² où R² représente :
1) un atome d'hydrogène ou un cation pharmacologiquement acceptable ;
2) un groupe alkyle à chaîne linéaire contenant 1 à 12 atomes de carbone, ou un groupe alkyle ramifié contenant 3 à 14 atomes de carbone ;
3) -Z-R³
où Z représente une liaison de valence ou un groupe alkylène à chaîne linéaire ou ramifié représenté par CₜH₂ₜ, où t représente un nombre entier de 1 à 6, et R³ représente un groupe cycloalkyle contenant 3 à 12 atomes de carbone, ou un groupe cycloalkyle substitué contenant 3 à 12 atomes de carbone et 1 à 3 substituants R⁴, chacun représentant un atome d'hydrogène ou un groupe alkyle contenant 1 à 5 atomes de carbone ;
4) - (CH₂CH₂O)ₙCH₃
où n est un nombre entier de 1 à 5;
5) -Z-Ar¹
où Z est défini comme ci-dessus, et Ar¹ représente un groupe phényle, α-naphtyle, β-naphtyle, 2-pyridyle, 3-pyridyle, 4-piridyle, α-furyle, β-furyle, α-thiényle, β-thiényle ou phényle substitué (où un substituant est au moins un parmi un atome de chlore, de fluor, d'iode, un groupe trifluorométhyle, alkyle contenant 1 à 4 atomes de carbone, nitro, cyano, méthoxy, phénoxy, p-acétamidobenzamide, -CH=N-NH-C (=O) -NH₂, -NH-C (=O) -Ph, -NH-C(=O)-CH₃ et -NH-C(=O)-NH₂) ;
6) -CₜH₂ₜCOOR⁴
où CₜH₂ₜ et R⁴ sont tels que définis ci-dessus ;
7) -CₜH₂ₜN(R⁴)₂
où C₂H₂ₜ et R⁴ sont tels que définis ci-dessus :
8) -CH(R⁵)-C(=O)-R⁶
où R⁵ représente un atome d'hydrogène ou un groupe benzoyle, et R⁶ représente un groupe phényle, p-bromophényle, p-chlorophényle, p-biphényle, p-nitrophényle, p-benzamidophényle ou 2-napthyle ;
9) -CₚH₂ₚ-W-R⁷
où W représente -CH=CH-, -CH=CR⁷ ou -C C-, et R⁷ représente un atome d'hydrogène ou un groupe alkyle ou aralkyle à chaîne linéaire ou ramifié contenant 1 à 30 atomes de carbone, et p représente un nombre entier de 1 à 5 ; ou
10) -CH (CH₂OR⁸)₂
où R⁸ représente un groupe alkyle ou acyle contenant 1 à 30 atomes de carbone ;
(B) -CH₂OH ;
(C) -C (=O)N(R⁹)₂
où R⁹ représente un atome d'hydrogène, un groupe alkyle à chaîne linéaire contenant 1 à 12 atomes de carbone, alkyle ramifié contenant 3 à 12 atomes de carbone, cycloalkyle contenant 3 à 12 atomes de carbone, cycloalkylalkylène contenant 4 à 13 atomes de carbone, phényle, phényle substitué (dans lequel le substituant est tel que défini dans (A)5)), aralkyle contenant 7 à 12 atomes de carbone, ou - SO₂R¹⁰, où R¹⁰ représente un groupe alkyle contenant 1 à 10 atomes de carbone, cycloalkyle contenant 3 à 12 atomes de carbone, phényle, phényle substitué (le substituant étant tel que défini dans (A)5)), ou aralkyle contenant 7 à 12 atomes de carbone, deux groupes R⁹ pouvant être identiques ou différents, et lorsque l'un des groupes R⁹ représente -SO₂R¹⁰, l'autre R⁹ ne représente pas -SO₂R¹⁰ ; ou
(D) -CH₂OTHP (THP représente un groupe tétrahydropyranyle) ;
A représente l'un des groupes suivants :
1) -(CH₂)ₘ-
2) -CH=CH-CH₂- ;
3) -CH₂-CH=CH- ;
4) -CH₂-O-CH₂-;
5) -CH=CH- ;
6) -O-CH₂-; ou
7) -C C- ;
où m représente un nombre entier de 1 à 3 ;
Y représente un atome d'hydrogène, un groupe alkyle contenant 1 à 4 atomes de carbone, un atome de chlore, de brome, de fluor, un groupe formyle, méthoxy ou nitro ;
B représente -X-C(R¹¹) (R¹²)OR¹³
où R¹¹ représente un atome d'hydrogène, un groupe alkyle contenant 1 à 4 atomes de carbone ; R¹³ représente un atome d'hydrogène, un groupe acyle contenant 1 à 14 atomes de carbone, aroyle contenant 6 à 15 atomes de carbone, tétrahydropyranyle, tétrahydrofuranyle, 1-éthoxythiényle ou t-butyle ; X représente l'un des suivants :
1) -CH₂-CH₂- ;
2) -CH=CH- ; ou
3) -C C- ; et
R¹² représente l'un des groupes suivants :
1) un groupe alkyle à chaîne linéaire contenant 1 à 12 atomes de carbone, ou alkyle ramifié contenant 3 à 14 atomes de carbone ;
2) -Z-Ar²
où Z est tel que défini ci-dessus, et Ar² représente un groupe phényle, α-naphtyle, β-naphtyle, ou phényle substitué par au moins un parmi un atome de chlore, de brome, de fluor, d'iode, un groupe trifluorométhyle, alkyle contenant 1 à 4 atomes de carbone, nitro, cyano, méthoxy, phényle ou phénoxy
3) -CₜH₂ₜOR¹⁴
où CₜH₂ₜ est tel que défini ci-dessus et R¹⁴ représente un groupe alkyle à chaîne linéaire contenant 1 à 6 atomes de carbone, alkyle ramifié contenant 3 à 6 atomes de carbone, phényle, phényle substitué par au moins un parmi un atome de chlore, de brome, de fluor, d'iode, un groupe trifluorométhyle, alkyle contenant 1 à 4 atomes de carbone, nitro, cyano, phényle ou phénoxy, cyclopentyle, cyclohexyle,
ou cyclopentyle ou cyclohexyle substitué par 1 à 4 groupes alkyle à chaîne linéaire contenant 1 à 4 atomes de carbone ;
4) -Z-R³
où Z et R³ sont tels que définis ci-dessus ;
5) -CₜH₂ₜ-CH=C (R¹⁵) R¹⁶
où CₜH₂ₜ est tel que défini ci-dessus et R¹⁵ et R¹⁶ représentent chacun un atome d'hydrogène, un groupe méthyle, éthyle, propyle ou butyle ; ou
6) -CᵤH₂ᵤ-C C-R¹⁷
où u est un nombre entier de 1 à 7, CᵤH₂ᵤ représente un groupe alkyle à chaîne linéaire ou ramifié, et R¹⁷ représente un groupe alkyle à chaîne linéaire contenant 1 à 6 atomes de carbone ;
E représente un atome d'hydrogène ou -OR¹⁸
où R¹⁸ représente un groupe acyle contenant 1 à 12 atomes de carbone, aroyle contenant 7 à 15 atomes de carbone, ou R² (où R² est tel que défini ci-dessus) ; et
la formule représente une forme d, l ou dl].

2. Utilisation selon la revendication 1, dans laquelle le dérivé de 4,8-inter-m-phénylène prostaglandine I₂, est le beraprost ou l'un de ses sels.

3. Utilisation selon la revendication 1 ou 2, dans laquelle la chimiokine C-C est MCP-1 ou MCP-3.

4. Utilisation selon les revendications 1 à 3, dans laquelle les maladies qui peuvent être guéries par un inhibiteur de production de chimiokine c-c sont des maladies **caractérisées par** une accumulation ou une activation anormale des leucocytes.

5. Utilisation selon les revendications 1 à 4, dans laquelle les leucocytes présentant une accumulation ou une activation anormale sont des monocytes et/ou des macrophages.

6. Utilisation selon les revendications 1 à 5, dans laquelle les leucocytes présentant une accumulation ou activation anormale comprennent au moins un type parmi les éosinophiles, les basophiles et les lymphocytes.
